# EUROPEAN PATENT SPECIFICATION

(11) **EP 4 284 295 B1**
(45) Date of publication and mention of the grant of the patent: **18.12.2024**
(21) Application number: 22704949.1
(22) Date of filing: 26.01.2022
(51) Int. Cl.: A61C 8/00, A61L 27/06

(54) **PROCESS FOR TREATING THE SURFACE OF AN IMPLANT MADE OF TITANIUM**
VERFAHREN ZUR OBERFLÄCHENBEHANDLUNG EINES IMPLANTATS AUS TITAN
PROCÉDÉ DE TRAITEMENT DE LA SURFACE D'UN IMPLANT EN TITANE

(30) Priority: 27.01.2021 IT 202100001574
(43) Date of publication of application: 06.12.2023
(73) Proprietor: Biomec S.r.l., 23823 Colico (LC) (IT)
(72) Inventor: DELLA BELLA, Olivio, 23823 Colico (Lecco) (IT); MOTTA, Francesca, 23017 Morbegno (Sondrio) (IT); BARILANI, Oscar, 23027 Samolaco (Sondrio) (IT); MUSSANO, Federico Davide, 10139 Torino (IT); GATTI, Giorgio, 17052 Borghetto Santo Spirito (Savona) (IT); D'ANGELO, Domenico, 10129 Torino (IT)
(74) Representative: Aseglio-Gianinet, Romina
(86) International application number: PCT/IB2022/050664
(87) International publication number: WO 2022/162542

(56) References cited:
- CN-A- 111 728 726
- JP-A- H08 257 109
- US-A1- 2017 151 004

## Description

Known materials in the field of dental implantology that are used for the rehabilitation of edentulous maxillary jaws have progressed a lot in terms of osseointegration, to the benefit of healing times and long-term success rates. In order to ensure high clinical standards, any material used for biological purposes, typically referred to as a biomaterial, has to be biocompatible, i.e. must not cause the death of cells, chronic inflammation or other damage to cellular and tissue functions. To this end, the biomaterials have to be selected so as to comply with three fundamental criteria: lack of cytotoxicity, lack of degradation, and structural suitability. Therefore, each aspect of a dental implant, from the design to the structure of the bulk, has to be adapted to the properties of the tissue to be replaced. In addition to the above requirements associated with the mass composition, the biocompatibility of the surfaces directly exposed to the biological medium is also extremely important. In order to promote the best interaction and integration with the tissue, it is necessary to have both the best chemical-physical compatibility at the tissue-implant interface and the best compatibility in terms of physical properties such as the surface roughness and texture. The development of studies in this field has led to the definition of behaviors that are influenced by structural hierarchies.

US2017151004 discloses a bone implant and a manufacturing method thereof. The bone implant includes an implanting body and a plurality of microstructures formed on a surface of the implanting body with an inner layer of the bone implanting body made of metal including titanium or titanium alloy, and an outer layer of the bone implanting body made of titanium dioxide.

The correct implementation of the implants needs to take into account the dimensional structure of the tissue that extends over approximately eight orders of magnitude, from the molecular scale of the cell adhesion receptors incorporated in the cell membrane (which interact with the proteins or the extracellular surfaces) up to the larger structures such as the tissues that have macroscopic dimensions.

An implant therefore has to be conceived and designed taking into account both the microscopic aspect and macroscopic aspect. In particular, dental implants made of titanium have a triple structural value which combines two levels: the microscopic level (primary chemical structure linked to the type of material and its roughness) and macroscopic level (linked to the texture, i.e. design, of the surface).

The primary chemical structure of the materials on the scale of the length of the bonds may be significantly influenced by the choice of material, i.e. may be based on ionic, covalent or metallic bonds from which secondary intramolecular effects such as electrostatic, H bond, van der Waals or hydrophobic interactions may be produced. The compatibility of an implant material is heavily determined by the primary chemical structure since, for example, properties such as resistance to corrosion, strength, resistance to wear, flexibility or solubility in water largely depend on the molecular arrangement.

The mass structure (1-100 nm) of the materials is responsible for the interaction between the tissue and the surface of the implants, and plays a fundamental role in regeneration. The structure on the micrometric level (> 1 micrometer) defines a further variable in the correlation between the influences of the length scale on the properties of the material and the effect on the tissue. The effects on the size of the grains may influence the strength, ductility or resistance to wear.

Both synthetic materials and biological systems have relevant functionalities over large length scales, thus allowing a wide range of adaptability for biomedical materials. Typically, higher-order structures such as the microstructure strictly dictate the kinetic processes and mechanical responses. In early hard tissue implant materials, the mass properties of these systems were generally taken into account (for example Young's modulus, tensile stress, resistance to fatigue, rigidity). Since then, global research in this field has laid the foundations for more advanced design of implant materials, on the basis of considerably improved understanding of natural materials and the material-biological organism interface.

The demand for materials for metal implants is characterized by a number of clinical studies. High mechanical strength is sought in order to ensure good load transmission over the long term and mechanical rigidity close to the bone. The corrosion resistance of the materials in the living organism is one of the main requirements in order to avoid deterioration of the properties of the materials as a result of degradation. Moreover, biocompatibility has to be ensured in order to prevent any damage to the host tissue that could be caused by the release of corrosion products or abrasive particles.

Until now, the materials most used commercially for titanium-based implants have been pure titanium and Ti6A14V. However, there has been some concern with regard to the high elastic modulus of these alloys by comparison with the bone (10-30 GPa). Commercially pure titanium is also selected for applications in which, in addition to its mechanical properties, resistance to corrosion is of paramount importance; these are essential in dental applications. The mechanical behavior, the chemical stability and the microstructure of the α-β Ti6Al4V alloy may be altered by thermal treatment or mechanical processing.

A recent trend in the research and development of titanium alloys specifically for biomedical applications takes into account the concerns regarding the toxic effects of the dissolution of the aluminum and vanadium ions in the host tissue that is a result of the wear caused by the corrosion of Ti6A14V. Recently, new titanium alloy compositions have been developed which are specifically intended for biomedical applications. These orthopedic alloys include Ti6Al7Nb and Ti5A12.5Fe, which are two alloys that have similar properties to Ti6Al4V and that have been developed in response to concerns about vanadium in terms of its potential cytotoxicity and adverse reactions with the host tissue.

A common aim in this field is to develop single-phase β-titanium alloys which are composed of non-toxic and hypoallergenic elements and which have excellent mechanical properties and good workability. A further advantage is that these β-phase titanium alloys exhibit an elastic modulus that is inherently lower than α-phase materials.

One important aspect concerns the fracture of the metal materials of the implant, and in particular fatigue fracture is considered to be the crucial problem among the various types of fracture. The characteristics of fatigue are closely correlated to the microstructure of the metal phases and thus also to the processing and heat treatments used. The fatigue characteristics of metal structural biomaterials must therefore always be considered for microstructures or specific processing parameters.

If the chemical composition of the materials is particularly relevant for obtaining a high degree of compatibility between the implant and the host tissue, other key determining factors are correlated to the surface, which is the actual interface with the biological environment. In addition to chemical composition, the various parameters of the surface that influence the response of the host tissue include wettability, roughness, electric charge, crystallinity and mobility.

There is also an affinity factor with the biological medium that is strongly determined by the combination of the chemical aspects of the surfaces and the morphological aspects thereof. In fact, the surface phenomena are mainly driven by a reduction associated with the free surface energy, resulting in greater chemical reactivity. At the same time, with the same predisposing chemical factors, the morphological component may clearly influence favorable interaction with the biological medium.

With regard to chemical aspects, the metal materials used in biomedical applications demonstrate the ability to spontaneously form a stable self-protective oxide layer (the so-called passive film) on the surface when reacting with air or more aqueous environments. This is, for example, typical behavior of titanium and its alloys, which ultimately leads to the surface formation of TiO₂.

Passive films have thicknesses of a few nanometers which provide highly effective barrier functions against the biological environment. The protective quality of a passive film is kinetically determined by the transfer of ions through the film and by the chemical stability thereof with respect to the biological environment. A series of factors such as the chemical composition, the structure, the thickness and the presence of defects therefore influence ionic transfer through the film and the stability of the film in various environments.

It is important to note that passivation reactions involve electrochemical steps, the patterns of which may be divided into intervals. There is the active interval, which corresponds to active dissolution (AD), a transition interval and a pre-passive interval (PPT), and a range in which it is possible to observe the formation of the passive layer (P).

The transition phase toward the formation of the passive layer includes the progressive covering of M(OH)x until the passivation potential has been reached, which is the point at which the surface is completely covered with adsorbates and deproteinization leads to the formation of the primary passivation film which mainly consists of MOx species. Therefore, given the operating conditions to which the metal and its alloys are exposed, it is important to protect the surface, with the dual function of increasing the durability of the biological environment (biostability) and maximizing its integration with the host tissues.

Important factors in terms of surface engineering include the impact of the chemistry of the surface, the topography on the micro and nanometric level, physical-chemical effects and biological factors, such as biochemically mediated cellular differentiation, the inevitable bacterial colonization of the implant, biological dimensions and histology of the surrounding structures.

Suitable surface modification techniques not only maintain the mass characteristics of the biomedical materials, but also improve the specific surface properties that are required by various clinical applications.

The influence of the surface roughness on the osseointegration rate and on the biomechanical fixation of the implants to the tissue has been identified as a key factor. Notably, surface topographies at the micrometric level have been identified as important, and various techniques for modifying the surfaces that function at this length scale have been developed. In particular, the observation of rapid and increased formation of bone contact by micrometer-scale roughened surfaces produced by sandblasting and subsequent acid-etching has produced considerable activity.

This has led to the conclusion that not only were suitably treated titanium implants entirely bio-inert or biocompatible, but also that suitable surface conditioning thereof influenced the adsorption of proteins, cellular activity and tissue response, i.e. all of the basic factors for achieving a higher level of osseointegration. Moreover, various works have demonstrated that the morphological characteristics on the micrometric scale control the speed and the quality of the formation of new tissue at the interface.

Schwartz et al. (Schwartz Z, Kieswetter K, Dean DD, Boyan BD. Underlying mechanisms at bone-surface interface during regeneration. J Periodontal Res. 1997 Jan; 32 (1 Pt2): 166-71. DOI: 10.1111/j. 1600-0765.1997.tb01399.x) report on the influence of surface roughness on titanium in order to influence the production of local factors involved in bone formation by osteoblasts, suggesting that the complement of autocrine and paracrine factors produced by the cells at the bone-implant interface may be directed by altering the roughness of the surface of the implant.

Numerous other studies have led to the conclusion that an optimal roughness for hard tissue implants is between 1 and 10 µm (D Duraccio, F Mussano, MG Faga Biomaterials for dental implants: current and future trends, Journal of Materials Science 50 (14), 4779-4812). It has been concluded that this roughness range exhibits the ability to maximize the interlocking between the mineralized bone and the surface of the implant.

In order to obtain osseointegrated implants, it is necessary for these to reach primary stability, i.e. their optimal mechanical coupling to the host tissue. It is not only the geometric requirements and the stress distribution factors of the host tissue that guide the topographical requirements on the surface of the implant, but also the consideration that the bone adapts to the mechanical load on account of the osteocytes which act as mechanosensors.

In order to meet the requirements for improved contact with the bone, various methods are used to create and stabilize these microstructural characteristics of the surface of the implant. These methods include sandblasting, acid engraving, anodization and plasma spraying.

Therefore, this invention relates in particular to a process for treating the surface of endo-osseous implants made of titanium and its alloys.

As is known, these "medical grade" titanium surfaces are prepared by means of processes which are typically divided into three to four steps. The first step usually consists of sandblasting, which aims to engrave the surface of the implant by utilizing the ablation produced by hard particles (typically alumina, titanium or hydroxyapatite). These are hurled at high pressure against the surfaces in order to make the surfaces smoother, remove surface pollutants and create an initial macro-roughness. The type of roughness may be modulated by suitably selecting the size of the particles.

Then, in the second step, an actual roughening treatment is carried out by means of acids (etching) in order to achieve the desired surface morphology. The acid treatment may be carried out using various combinations which have been established on the basis of acquired data relating to biocompatibility and osseointegration tests, as well as the large amount of data on the surface-property correlations that has been acquired over the years. The treatment includes a first etching step using hydrofluoric acid, followed by a second sulfuric-phosphoric acid etching. Other treatments differ in terms of the type of acids used. Often, the necks of implants such as screws are hidden in order to prevent them from being subjected to etching. This differentiation is due to the need to protect this zone from etching, because this zone, if it had a micro-roughness, could promote bacterial colonization once implanted.

The samples treated in this way are then dried in stages and cleaned by means of a sequence of treatments using solvents (third step). In some cases, a surface treatment process is carried out using cold plasma of Ar or Ar and O₂ (fourth step). Although this process gives excellent results in terms of the effectiveness of the devices during the implantation stage, it has several problems during the preparation stage, which problems range from the need to dispose of considerable quantities of acid to the impossibility of providing large supplies while maintaining optimal qualitative standards.

The surface treatments that are most commonly used commercially ensure a roughness having a Sa value in the range from 0.3 to 3 mm, which the most precise authors further subdivide into minimum roughness (0.5-1 mm) and moderate roughness (1-2 mm), reserving the classification of "maximum" roughness for values of Sa greater than 2 mm and smooth surfaces as being those which have values of Sa less than 0.4 mm.

Therefore, an object of this invention is to provide a process for treating the surface of implants which are made of titanium and have variable dimensions and geometries, which process makes it possible to obtain a desired roughness and surface texture as well as controlled oxidation of the surface.

This object is achieved by means of a process for treating a surface of an implant made of titanium or its alloys, in particular an endo-osseous implant for oral cavity or orthopedic applications, the process comprising the successive steps of:
- treating said surface with laser radiation coming from a nano- or femtofiber laser source, in such a way that said surface is ablated and assumes a predetermined texture, and
- treating said surface with plasma jet at atmospheric pressure, using a process gas containing air, in such a way that a layer of titanium oxide up to 30 nm thick is formed on said surface.

In preferred embodiments of the invention, the process is carried out on implants made of ASTM F67 grade 4 titanium, in particular endo-osseous screws which have a length in the range of 5 to 60 mm, a thread with an intercrestal distance in the range of 0.2 to 2.5 mm, and a diameter in the range of 1.8 to 8 mm.

The step of treatment with laser radiation advantageously generates a lattice pattern of a plurality of hemispherical wells on the surface of the implant. Said lattice pattern preferably has a mesh size in the range of 15 to 50 µm, and said hemispherical wells have a depth in the range of 10 to 18 µm and a diameter in the range of 10 to 50 µm.

The process of the invention makes it possible to effectively control both the "roughness," meaning as such the whole of the irregularity and discontinuity in the profile of the surface, and the "pattern," i.e. the regular geometric distribution of micro-cavities such as the aforementioned hemispherical wells.

The process of the invention has been proven to be highly efficient as a result of the combination of laser and cold atmospheric plasma technologies, making it possible to obtain both a micro-roughness which is controlled and effective in terms of osseointegration and a chemical-physical composition which is similar to the biological environment.

In its first step, the process of the invention makes it possible to create a texture on the surface of the implant as a result of the laser radiation emitted by a nano- or femtofiber laser source which advantageously has a focal length of 100 or 160 mm, a frequency between 50 and 130 kHz, and a power between 5 and 50 W.

One usable laser device is manufactured, for example, by GF Machining Solutions (Geneva, Switzerland), and allows effective focusing of the beam through a controlled movement over 5 axes.

The frequency of repetition of the pulse varies from a single pulse to 130 kHz. The energy of the pulsed laser used is in the range between 1.4 mJ and 2.0 mJ, and the scanning speed of the laser is in the range of 26 to 157 mm/s. By setting the parameters Sa, Sku, Smean, Sp, Sq, Ssk, Sv, Sz, Sdp Sdr, Sal and Str as desired, it is possible to obtain a roughness and pattern on the surface of the implant that maximize the optimal mechanical coupling between this surface and the mineralized bone.

In the second step of the process of the invention, a layer of titanium oxide, in particular TiO₂, is formed on the surface of the implant by means of plasma technology. For example, an arc plasma source (1000 W) may be used which operates at a distance in the range of 7 to 12 mm from the surface of the implant and a frequency in the range of 19 to 25 kHz.

The second oxidative step of the process of the invention makes it possible to remove the pollutant agents inherently present on the surface of the implant and residues left by the microfusion processes carried out during the first laser step.

In preferred embodiments of the process of the invention, the process gas used in the second step is air or an air/H₂O mixture or an air/H₂O₂ mixture, while the oxide layer produced on the surface of the implant is typically TiO₂ and has a thickness in the range of 7 to 20 nm.

This final surface chemical composition allows for more flexible interaction with the biological environment, thus increasing the reaction kinetics between the latter and the surface of the implant, with the added result of increasing durability in the biological environment (biostability) and maximizing the integration of the implant with the tissues that host it.

Further advantages and features of this invention will become evident from the following embodiments provided by way of non-limiting example and with reference to the accompanying drawings, in which:
Fig. 1 is a series of photographs, on a gradually increasing scale, of a laser-treated surface according to example 1,
Fig. 2 is a series of photographs, on a gradually increasing scale, of a plasma-treated surface according to example 1,
Fig. 3 is a series of photographs, on a gradually increasing scale, of a surface treated with laser and plasma according to example 1 and then used as a substrate for cell growth,
Fig. 4 is a series of photographs, on a gradually increasing scale, of a laser-treated surface according to example 2,
Fig. 5 is a series of photographs, on a gradually increasing scale, of a plasma-treated surface according to example 2,
Fig. 6 is a series of photographs, on a gradually increasing scale, of a surface treated with laser and plasma according to example 2 and then used as a substrate for cell growth,
Fig. 7 is a series of photographs, on a gradually increasing scale, of a laser-treated surface according to example 3,
Fig. 8 is a series of photographs, on a gradually increasing scale, of a plasma-treated surface according to example 3, and
Fig. 9 is a series of photographs, on a gradually increasing scale, of a surface treated with laser and plasma according to example 3 and then used as a substrate for cell growth.

### EXAMPLE 1

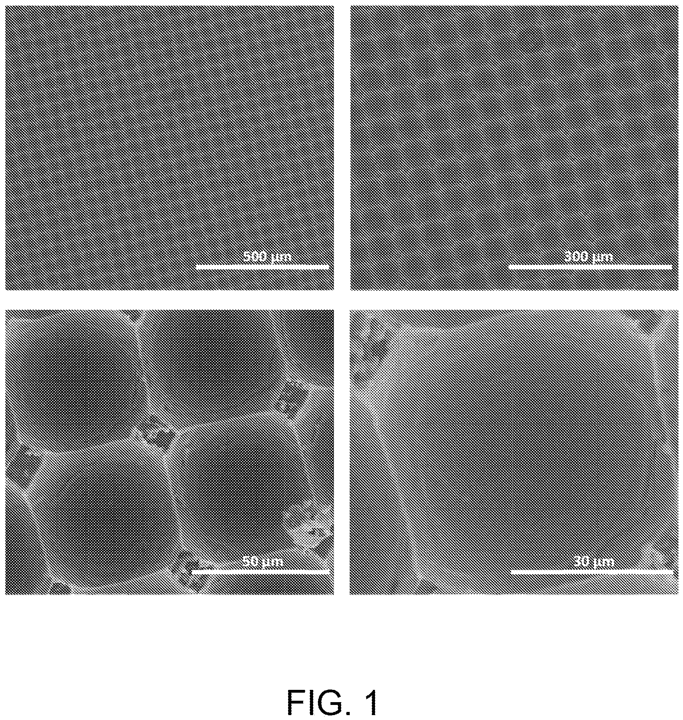

First, three series of implants made of ASTM F67 grade 4 titanium and having dimensions L (length) 10.00 mm, F (intercrestal distance of the thread) 0.9 mm and D (diameter) 4 mm were surface-treated (step 1) using a nanofiber laser (30 W) with focal lens 160 mm, 4 repetitions, power 50%, frequency 50 kHz, points distance 25 µm, and depth 18 µm (Fig. 1).

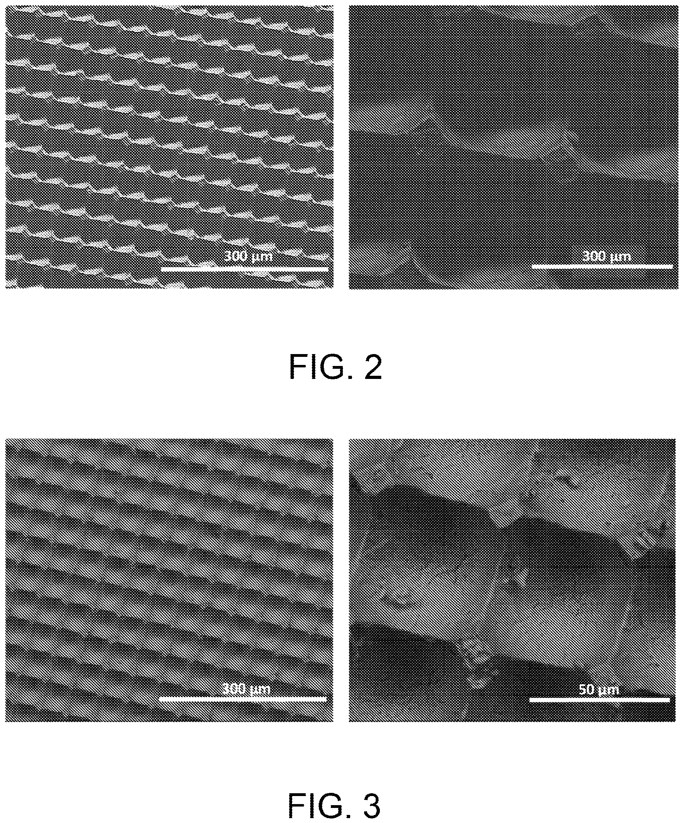

The surfaces ablated by the laser were then subjected to an arc plasma treatment which operates at a distance of 11 mm between the nozzle and the surface of the implant, power 665 W and frequency 25 kHz and which uses, as a process gas, air for series 1, an air/H₂O mixture for series 2 and an air/H₂O₂ mixture for series 3 (Fig. 2).

The series of implants treated in this way were then used as substrates for cell growth (Fig. 3).

### EXAMPLE 2

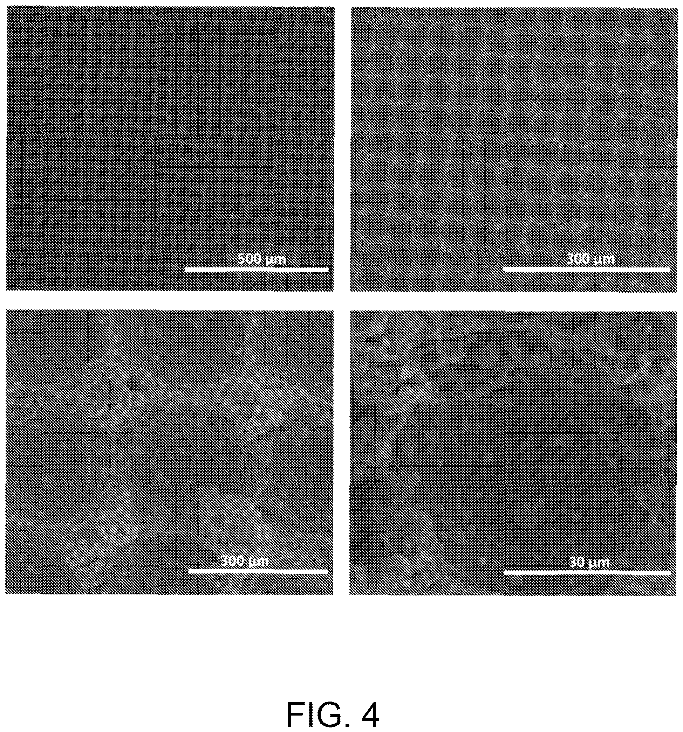

First, three series of implants made of ASTM F67 grade 4 titanium and having dimensions L (length) 10.00 mm, F (intercrestal distance of the thread) 0.9 mm and D (diameter) 4 mm were treated (step 1) using a nanofiber laser (30 W) with focal lens 160 mm, 4 repetitions, power 100%, frequency 130 kHz, points distance 50 µm, and depth 18 µm (Fig. 4).

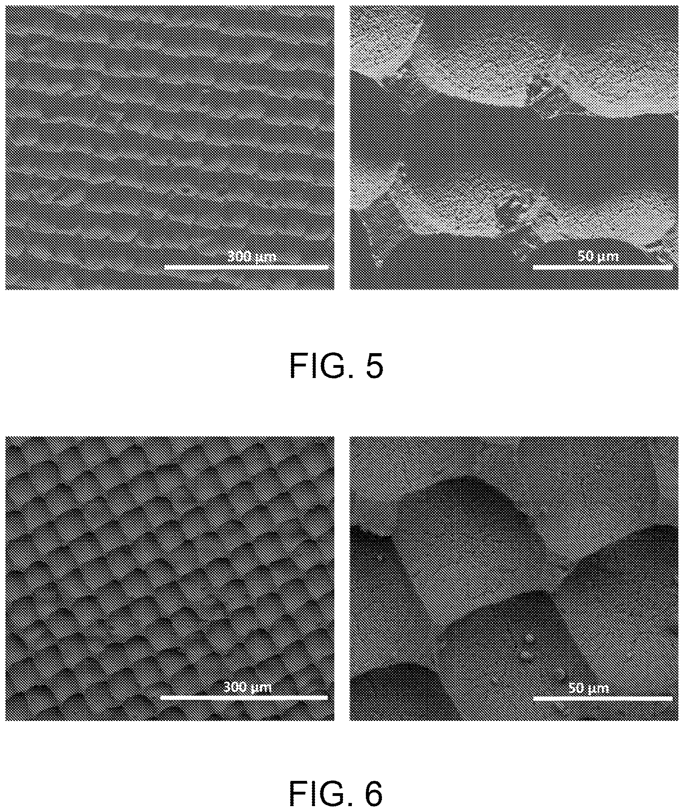

The surfaces ablated by the laser were then subjected to an arc plasma treatment which operates at a distance of 11 mm between the nozzle and the surface of the implant, power 665 W and frequency 25 kHz and which uses, as a process gas, air for series 1, an air/H₂O mixture for series 2 and an air/H₂O₂ mixture for series 3 (Fig. 5).

The series of implants treated in this way were then used as substrates for cell growth (Fig. 6).

### EXAMPLE 3

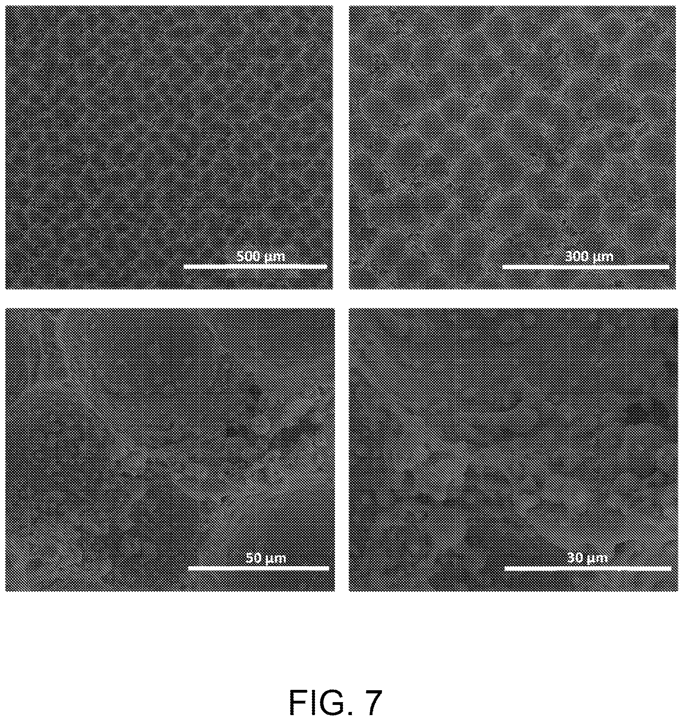

First, three series of implants made of ASTM F67 grade 4 titanium and having dimensions L (length) 10.00 mm, F (intercrestal distance of the thread) 0.9 mm and D (diameter) 3.5 mm were treated (step 1) using a nanofiber laser (30 W) with focal lens 160 mm, 4 repetitions, power 100%, frequency 130 kHz, points distance 50 µm, and depth 18 µm (Fig. 7).

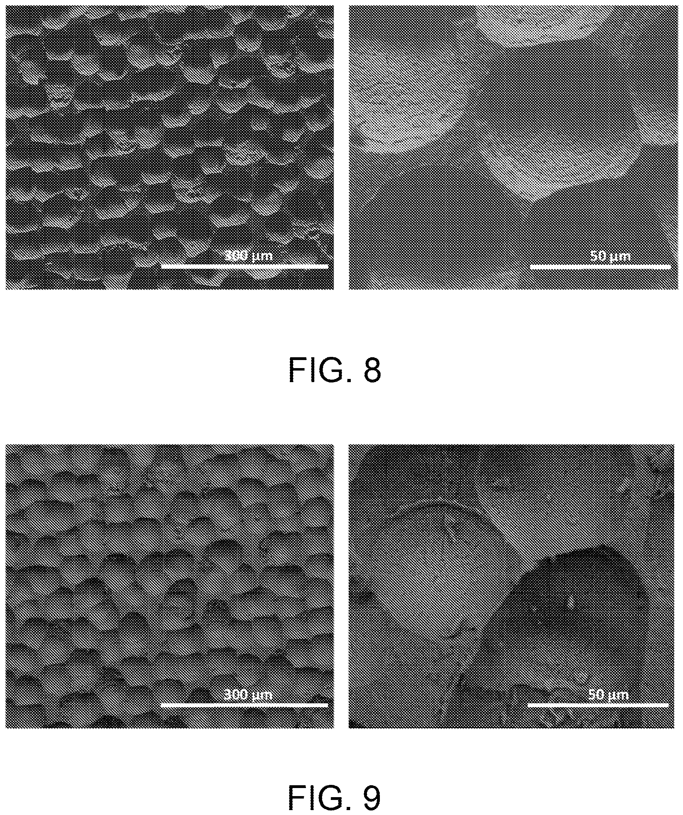

The surfaces ablated by the laser were then subjected to an arc plasma treatment which operates at a distance of 11 mm between the nozzle and the surface of the implant, power 665 W and frequency 25 kHz and which uses, as a process gas, air for series 1, an air/H₂O mixture for series 2 and an air/H₂O₂ mixture for series 3 (Fig. 8).

The series of implants treated in this way were then used as substrates for cell growth (Fig. 9).

### EXAMPLE 4 (COMPARISON)

In order to demonstrate the advantages of the process of the invention, comparative implants were surface-treated in a conventional manner, i.e. with preliminary sandblasting and subsequent acid-etching.

The series of implants treated in the conventional manner were then used as substrates for cell growth and then subjected to various tests.

In detail, the implants of the examples described above were subjected to an immediate cell adhesion test which was evaluated by fluorescence microscopy by counting the nuclei of the cells adhered to each sample. Three technical repeats were carried out for each experiment. The statistical analysis was carried out by means of ANOVA, taking into consideration a p-value of < 0.05.

The implants of the examples described above were also subjected to a cell proliferation (vitality) test which was evaluated by luminometric assay of the production of ATP, which is correlated to the number of cells. Three technical repeats were carried out for each experiment. The statistical analysis was carried out by means of ANOVA, taking into consideration a p-value of < 0.05.

Moreover, the implants of the examples described above were subjected to a test for the production of osteocalcin, which made it possible to evaluate the surfaces with the highest production of osteocalcin released from the cells which were kept for 4 weeks in an osteoinductive culture medium, by metering using ELISA kits in starvation media collected according to the methodology described in Mussano et al. (Int. J. Mol. Sci. 14 May 2018; 19(5). PII: E1454. DOI: 10.3390/ijms19051454), by comparison with the positive control. Three technical repeats were carried out for each experiment. The statistical analysis was carried out by means of ANOVA, taking into consideration a p-value of < 0.05.

Specifically, the following results were obtained.

### Cell adhesion

1) The implants in the comparative example demonstrated cell adhesion (precursor to osteoblasts) equal to 170 cells/implant.
2) The implants in series 3 in example 1 (air/H₂O₂ mixture) demonstrated cell adhesion equal to 280 cells/implant (precursor to osteoblasts), which is much higher than that of the implants in the comparative example.
3) The implants in series 3 in example 2 (air/H₂O₂ mixture) demonstrated cell adhesion of 240 cells/implant (precursor to osteoblasts), which is also higher than that of the implants in the comparative example.

### Cell proliferation

1) The implants in the comparative example demonstrated levels of cell proliferation, measured using commercial chemiluminescent kits, equal to 24,000 RLU.
2) The implants in series 3 in example 1 (air/H₂O₂ mixture) demonstrated cell proliferation equal to 31,000 RLU, which is much higher than that of the implants in the comparative example.
3) The implants in series 3 in example 2 (air/H₂O₂ mixture) demonstrated cell proliferation equal to 30,000 RLU, which is much higher than that of the implants in the comparative example.

### Production of osteocalcin

1) The implants in the comparative example demonstrated doses of osteocalcin equal to 90 pg/ml.
2) The implants in series 3 in example 1 (air/H₂O₂ mixture) demonstrated doses of osteocalcin equal to 115 pg/ml, which is higher than that of the implants in the comparative example.
3) The implants in series 3 in example 1 (air/H₂O₂ mixture) demonstrated doses of osteocalcin equal to 140 pg/ml, which is much higher than that of the implants in the comparative example.

Of course, the details of implementation and the embodiments may vary widely with respect to that which has been described purely by way of example, without thereby departing from the scope of the invention as defined in the appended claims.

## Claims

1. A process for treating a surface of an implant made of titanium or its alloys, comprising the successive steps of:
- treating said surface with laser radiation coming from a nano- or femtofiber laser source, in such a way that said surface is ablated and assumes a predetermined texture, and
- treating said surface with plasma jet at atmospheric pressure, using a process gas containing air, in such a way that a layer of titanium oxide up to 30 nm thick is formed on said surface.

2. The process according to claim 1, wherein said fiber laser source is of the nano type and has a power in the range of 5 to 50 W, preferably 30 W.

3. The process according to claim 1, wherein said fiber laser source is of the femto type and has a power in the range of 5 to 50 W, preferably 20 W.

4. The process according to any one of the preceding claims, wherein said process gas is selected from the group consisting of air, an air/H₂O mixture and an air/H₂O₂ mixture.

5. The process according to any one of the preceding claims, wherein said implant is made of ASTM F67 grade 4 titanium or its alloys.

6. The process according to any one of the preceding claims, wherein said step of treatment with laser radiation generates a lattice pattern of a plurality of preferably hemispherical wells on said surface.

7. The process according to claim 6, wherein said lattice pattern has a mesh size in the range of 15 to 50 µm, and said hemispherical wells have a depth in the range of 10 to 18 µm and a diameter in the range of 10 to 50 µm.

8. The process according to any one of the preceding claims, wherein said titanium oxide is TiO₂.

9. The process according to any one of the preceding claims, wherein said implant is an endo-osseous screw.

10. The process according to claim 9, wherein said endo-osseous screw has a length in the range of 5 to 60 mm, a thread with an intercrestal distance in the range of 0.2 to 2.5 mm, and a diameter in the range of 1.8 to 8 mm.

11. The process according to any one of the preceding claims, wherein said implant is an endo-osseous implant, in particular for oral cavity or orthopedic applications.

## Patentansprüche

1. Verfahren zur Oberflächenbehandlung eines Implantats aus Titan oder dessen Legierungen, umfassend die folgenden aufeinanderfolgenden Schritte:
- Behandeln der Oberfläche mit Laserstrahlung aus einer Nano- oder Femtofaser-Laserquelle, sodass die Oberfläche abgetragen wird und eine vorbestimmte Textur annimmt, und
- Behandeln der Oberfläche mit einem Plasmastrahl bei atmosphärischem Druck, unter Verwendung eines Prozessgases, das Luft enthält, sodass eine Schicht aus Titanoxid mit einer Dicke von bis zu 30 nm auf der Oberfläche gebildet wird.

2. Verfahren nach Anspruch 1, wobei die Faserlaserquelle des Nano-Typs ist und eine Leistung im Bereich von 5 bis 50 W, vorzugsweise 30 W, aufweist.

3. Verfahren nach Anspruch 1, wobei die Faserlaserquelle des Femto-Typs ist und eine Leistung im Bereich von 5 bis 50 W, vorzugsweise 20 W, aufweist.

4. Verfahren nach einem der vorhergehenden Ansprüche, wobei das Prozessgas aus der Gruppe, die aus Luft, einem Luft/H₂O-Gemisch und einem Luft/H₂O₂-Gemisch besteht.

5. Verfahren nach einem der vorhergehenden Ansprüche, wobei das Implantat aus Titan Grade 4 ASTM F67 oder dessen Legierungen besteht.

6. Verfahren nach einem der vorhergehenden Ansprüche, wobei der Schritt des Behandelns mit Laserstrahlung ein Gittermuster aus einer Vielzahl von vorzugsweise halbkugelförmigen Vertiefungen auf der Oberfläche erzeugt.

7. Verfahren nach Anspruch 6, wobei das Gittermuster eine Maschengröße im Bereich von 15 bis 50 µm aufweist und die halbkugelförmigen Vertiefungen eine Tiefe im Bereich von 10 bis 18 µm und einen Durchmesser im Bereich von 10 bis 50 µm aufweisen.

8. Verfahren nach einem der vorhergehenden Ansprüche, wobei das Titanoxid TiO₂ ist.

9. Verfahren nach einem der vorhergehenden Ansprüche, wobei das Implantat eine enossale Schraube ist.

10. Verfahren nach Anspruch 9, wobei die enossale Schraube eine Länge im Bereich von 5 bis 60 mm, ein Gewinde mit einem Abstand zwischen den Gewindespitzen im Bereich von 0,2 bis 2,5 mm und einen Durchmesser im Bereich von 1,8 bis 8 mm aufweist.

11. Verfahren nach einem der vorhergehenden Ansprüche, wobei das Implantat ein enossales Implantat ist, insbesondere für Anwendungen in der Mundhöhle oder in der Orthopädie.

## Revendications

1. Un procédé de traitement de la surface d'un implant en titane ou en ses alliages, comprenant les étapes successives suivantes:
- traitement de ladite surface avec une radiation laser provenant d'une source laser à fibre de type nano ou femto, de telle sorte que ladite surface soit ablatée et présente une texture prédéterminée, et
- traitement de ladite surface avec un jet de plasma à pression atmosphérique, en utilisant un gaz de procédé contenant de l'air, de telle sorte qu'une couche d'oxyde de titane d'une épaisseur pouvant atteindre 30 nm soit formée sur ladite surface.

2. Le procédé selon la revendication 1, dans lequel ladite source laser à fibre est de type nano et a une puissance comprise entre 5 et 50 W, de préférence 30 W.

3. Le procédé selon la revendication 1, dans lequel ladite source laser à fibre est de type femto et a une puissance comprise entre 5 et 50 W, de préférence 20 W.

4. Le procédé selon l'une quelconque des revendications précédentes, dans lequel ledit gaz de procédé est choisi parmi le groupe constitué d'air, d'un mélange air/H₂O et d'un mélange air/H₂O₂.

5. Le procédé selon l'une quelconque des revendications précédentes, dans lequel ledit implant est en titane de grade 4 ASTM F67 ou en ses alliages.

6. Le procédé selon l'une quelconque des revendications précédentes, dans lequel ladite étape de traitement avec radiation laser génère un motif en treillis d'une pluralité de puits, de préférence hémisphériques, sur ladite surface.

7. Le procédé selon la revendication 6, dans lequel ledit motif en treillis a une maille de dimension comprise entre 15 et 50 µm, et lesdits puits hémisphériques ont une profondeur comprise entre 10 et 18 µm et un diamètre compris entre 10 et 50 µm.

8. Le procédé selon l'une quelconque des revendications précédentes, dans lequel ledit oxyde de titane est du TiO₂.

9. Le procédé selon l'une quelconque des revendications précédentes, dans lequel ledit implant est une vis endo-osseuse.

10. Le procédé selon la revendication 9, dans lequel ladite vis endo-osseuse a une longueur comprise entre 5 et 60 mm, un filet avec une distance intercrêtes comprise entre 0,2 et 2,5 mm, et un diamètre compris entre 1,8 et 8 mm.

11. Le procédé selon l'une quelconque des revendications précédentes, dans lequel ledit implant est un implant endo-osseux, en particulier pour des applications dans la cavité buccale ou orthopédiques.
